# EUROPEAN PATENT APPLICATION

(11) **EP 4 520 324 A1**
(43) Date of publication of application: **12.03.2025**
(21) Application number: 23315346.9
(22) Date of filing: 11.09.2023
(51) Int. Cl.: A61K 31/198, A61K 31/197, A61K 31/4409, A61K 45/06, A61P 35/00

(54) **COMPOSITION COMPRISING A TAURINE TRANSPORTER (TAUT) INHIBITOR AND 5-AMINOLEVULINIC ACID (5-ALA), ITS USE IN TREATING CANCER CELL TUMORS, AND ANALYSIS OF THE EFFECTIVENESS OF THE COMPOSITION**

(71) Applicant: Endress+Hauser Process Analysis Support SARL, 69800 Saint-Priest (FR)
(72) Inventor: Lambert Angulo, Leticia, 69970 Chaponnay (FR)
(74) Representative: Endress + Hauser Group Services (Deutschland) AG+Co. KG

(57) **Abstract**

The present invention provides a composition comprising a taurine transporter (TauT) inhibitor, selected from piperidine-4-sulfonic acid, g-Aminobutyric acid, g-vinyl-aminubutyric acid, β-alanine, or any pharmaceutically acceptable salt thereof, preferably the TauT inhibitor is piperidine-4-sulfonic acid or a pharmaceutically acceptable salt thereof and 5-aminolevulinic acid (5-ALA) or a pharmaceutically acceptable salt thereof, said composition for use in treating cancers with a mutated p53, as well as a method for analyzing the effective amount and ratio of a taurine transporter (TauT) inhibitor, selected from piperidine-4-sulfonic acid, g-Aminobutyric acid, g-vinyl-aminubutyric acid, β-alanine or any pharmaceutically acceptable salt thereof, preferably the TauT inhibitor is piperidine-4-sulfonic acid or a pharmaceutically acceptable salt thereof , and 5-aminolevulinic acid (5-ALA) or a pharmaceutically acceptable salt thereof to kill cancer cells by Raman spectroscopy.

## Description

One in five people worldwide will be diagnosed with cancer in their lifetime. Nowadays, chemotherapy is the standard cancer treatment (International Agency for Research on Cancer).

The TP53 gene, which encodes the p53 protein, which is also called Tumor protein P53, cellular tumor antigen p53, or transformation-related protein 53 (TRP53), is the most frequent target for mutation in tumors, with over half of all human cancers exhibiting mutation at this locus (Vogelstein et al., 2000). In response to a number of stressors, including DNA damage, hypoxia and oncogenic activation, p53 becomes activated to promote cell cycle arrest, apoptosis or senescence thereby suppressing tumor growth. It also plays many additional roles including regulating cellular metabolism (Muller et al., 2009). TP53 activation also initiates a number of DNA repair pathways (Fei and EI'Deiry, 2003, Oncogene 22:5774-83).

Mutations in TP53, which are present in about 50% of human cancers (Hollstein et al., 1991, Science.253:49-53), result in checkpoint defects and may contribute to uncontrolled cell proliferation, genomic instability, and accumulation of tumorigenic mutations (Prives and Hall, 1999, J. Pathol. 186:112-26). In cancer cells with a mutated p53 the tumor suppressor protein p73 is inactivated. Mouse double minute 2 homolog MDM2 or E3 ubiquitin-protein ligase is a negative regulator of the p53 tumor suppressor that binds to the inactive p73 and the mutant p53, whereby the three molecules form a complex. MDM2 is a negative regulator of p53 and p73. A further molecule, which attaches to p73 is the E3 ubiquitin-protein ligase ITCH. It ubiquitinylates p73 and thereby catalyses its degradation.

Nowadays, depending on the tumor, chemotherapy and/or radiotherapy are the standard cancer treatment. However, in many cases, it is ineffective when cancer cells develop resistance, or side effects occur from drug administration.

Furthermore, compositions, which are suitable for the treatment of brain tumors, are restricted, as the blood-brain-barrier prevents large protein-based pharmaceuticals, biologicals, from entering the brain. Therefore, there is a need for compositions, which pass through the blood-brain barrier for the treatment of brain tumors.

Thus, there is a need for compositions, which effectively destroy tumors, and have less or preferably have no adverse side effects of commonly used chemotherapeutics. Also, it is desired to provide compositions, which are able to pass the blood-brain-barrier to target brain tumors.

In cancers retaining intact TP53 gene, p53 protein may be targeted for degradation by the deregulated E3 ubiquitin ligase Mouse double minute 2 homolog or MDM2.

5-ALA (5-aminolevulinic acid) is an amino acid that is naturally present in the human body. 5-ALA is a precursor of a small molecule called protoporphyrin IX (PpIX), which is a small molecule, which can cross the blood-brain barrier. 5-ALA does not cause toxicity in normal cells.

TauT inhibitors exist, which are also capable of passing the blood-brain barrier. These components include piperidine-4-sulfonic acid, g-Aminobutyric acid, g-vinyl-aminobutyric acid, β-alanine, and GABA-mimetics such as gaboxadol or 4,5,6,7-tetrahydroisoxazolo(5,4-c)pyridin-3-ol (THIP), nipecotic acid, and guvacine or any pharmaceutically acceptable salt thereof. Further TauT inhibitor include polypeptide-based antibodies, as shown in US 20190202885 A1.

In both cancers - with and without a p53 mutation PpIX disintegrates the complex including MDM2 and mutated or intact p53, which leads to an improved regulation of cell proliferation.

In cancer cells with mutated p53, PpIX also interacts with p73, releasing p73 from MDM2, and hereby allowing p73 to reactivate its antitumor properties. Hence, PpIX triggers accumulation of p73, and inhibits its desintegrtion.

PpIX also fulfills a pro-oxidant function, by inhibiting thioredoxin reductase (TrxR) and elevating Reactive Oxygen Species (ROS) level for the induction of apoptosis (cell death) in cancer.

Elevating Reactive Oxygen Species (ROS) levels can be also achieved by the inhibition of the Taurine Transporter (TauT) - a membrane protein that transports taurine into the cell. Taurine is a natural antioxidant in the body. Lack of taurine has been shown to impair the transfer of electrons in mitochondria and to increase the production of free radicals. Besides, taurine also suppresses enzymes responsible for ROS generation. With no taurine stimulating antioxidant activity, there will be an increase in the ROS levels up to a point that mutant p53 cannot tolerate; thus, the cell being forced to undergo apoptosis.

Taurine T inhibitors such as piperidine-4-sulfonic acid, g-Aminobutyric acid, g-vinyl-aminobutyric acid β-alanine, or a pharmaceutically acceptable salt thereof are -like PpIX - small molecules, which passes the blood-brain barrier.

Mutant p53 is thus the common target of both PpIX and TauT inhibitors for killing cancerous cells by inducing apoptotic cell death.

The object of this invention is to provide an anti-tumor composition, which is less toxic than commonly used chemotherapeutic agents and which passes the blood-brain-barrier. Another aspect of the invention is the provision of a composition for use in treating cancer cells of a human. A further aspect of the invention is the provision of an analysis method, which allows for determining the effective concentration and ratio of the components required to reduce tumor growth, which may be applied in real-time, and which is conducted on living cells.

In a first aspect, the invention provides a composition, wherein the composition comprises:
(i) a taurine transporter (TauT) inhibitor, wherein preferably the TauT inhibitor is selected from
   piperidine-4-sulfonic acid, g-Aminobutyric acid, g-vinyl-aminubutyric acid, β-alanine, or any pharmaceutically acceptable salt thereof, preferably
   piperidine-4-sulfonic acid or a pharmaceutically acceptable salt thereof and
(ii) 5-aminolevulinic acid (5-ALA) or a pharmaceutically acceptable salt thereof.

A pharmaceutically acceptable salt of 5-ALA is , e.g., 5-Aminolevulinic acid hydrochloride.

In addition to the effect of disintegrating a p53/MDM2 complex by PpIX, a combination of a Taurine Transporter inhibitor and 5-ALA, which is converted into PpIX in cells, specifically in the mitochondrion, aims at enhancing the Reactive Oxygen Species (ROS). Thus, a combination of PpIX and Taurine Transporter inhibitor, such as piperidine-4-sulfonic acid or a pharmaceutically acceptable salt thereof, aims at forcing the cancer cells to undergo apoptosis

In one embodiment, the composition comprises a pharmaceutical carrier, wherein preferably the carrier is an adjuvant.

Adjuvats include state-in-the art adjuvants, which are used for active agents used for oral dosage forms.

The invention further provides a composition of the first aspect of the invention or an embodiment thereof for use in treating cancer, wherein the composition is to be administered to a cancer patient to inhibit the growth of cancer cells.

In one embodiment, the composition for the use comprising TauT inhibitor, selected from piperidine-4-sulfonic acid, g-Aminobutyric acid, g-vinyl-aminubutyric acid, β-alanine, or any pharmaceutically acceptable salt thereof, preferably the piperidine-4-sulfonic acid or a pharmaceutically acceptable salt thereof and 5-aminolevulinic acid (5-ALA) or a pharmaceutically acceptable salt thereof are to be administered orally, wherein the administration of TauT inhibitor or a pharmaceutically acceptable salt thereof and 5-aminolevulinic acid (5-ALA) or a pharmaceutically acceptable salt thereof is separate, sequential, and/or simultaneous.

In one embodiment, TauT inhibitor, selected from piperidine-4-sulfonic acid, g-Aminobutyric acid, g-vinyl-aminubutyric acid acid, β-alanine, or any pharmaceutically acceptable salt thereof, preferably
piperidine-4-sulfonic acid or a pharmaceutically acceptable salt thereof is to be administered to a human in a total amount of 500mg, and
5-aminolevulinic acid (5-ALA) or a pharmaceutically acceptable salt thereof is to be administered to a human in an amount of 100 mg.

In one embodiment, the piperidine-4-sulfonic acid, g-Aminobutyric acid, g-vinyl-aminubutyric acid, β-alanine, or a pharmaceutically acceptable salt thereof is administered twice daily and 5-aminolevulinic acid (5-ALA) or a pharmaceutically acceptable salt thereof is to administered in an amount of 100 mg once daily.

In one embodiment, the administration regimen is repeated for at least 2 months, preferably for 2-6 months.

In one embodiment, the orally to be administered composition is comprised in tablets or capsules.

In one embodiment, the administered composition is combined with readiotherapy or photodynamic therapy. Preferably, radiotherapy is carried out over a period of 6 weeks, wherein the radiotherapy is initiated in the beginning of the adminstration regimen.

In one embodiment, the cancer cells or tumor cells have a mutation in the TP53 gene, wherein the mutation results in an altered expression of a p53 translation product and/or in expression of a non-functional p53 protein that corresponds to an alteration in a human p53 gene associated with tumor growth or wherein the cancer cells retain intact TP53 gene
Preferably, the cancer cells are tumor cells that have a mutation in the TP53 gene, wherein the mutation results in an altered expression of a p53 translation product and/or in expression of a non-functional p53 protein that corresponds to an alteration in a human p53 gene associated with tumor growth.

In an alternative embodiment, the cancer cells retain an intact p53 gene.

In one embodiment the cancer is a brain tumor, in particular, a glioblastoma, a pancreatic tumor, a kidney tumor, colon carcinoma, osteosarcoma, or wherein the cancer is leukemia. Preferably, the cancer is a brain tumor, in particular a glioblastoma.

The invention further provides a kit for analyzing anti-tumor effect of 5-aminolevulinic acid and/or taurine transporter (TauT) inhibitor comprising
(i) a Raman spectrometer
(ii) cell imaging apparatus, and
(iii) a composition of the invention or an embodiment thereof or a composition for the use of the invention or an embodiment thereof.

The invention further provides an *in vitro* method for monitoring the efficacy of anti-tumor growth therapy of a composition according the first aspect of the invention or an embodiment thereof, wherein the method comprises:
(i) providing tumor cells as a cell culture having a mutation in the TP53 gene, wherein the mutation results in an altered expression of a p53 translation product and/or in expression of a non-functional p53 protein that corresponds to an alteration in a human p53 gene associated with tumor growth;
(ii) contacting the tumor cells with a taurine transporter (TauT) inhibitor, wherein the taurine transporter inhibitor is selected from
   piperidine-4-sulfonic acid, g-Aminobutyric acid, g-vinyl-aminubutyric acid, β-alanine, or a pharmaceutically acceptable salt thereof and/or
(iii) contacting the tumor cells with 5-aminolevulinic acid (5-ALA) or a pharmaceutically acceptable salt thereof
(iv) optionally contacting the tumor cells with an anti-cancer drug selected from a chemotherapeutic agent,
(v) and acquiring a Raman spectra of the cancer cell culture samples online.

Raman spectroscopy is a spectroscopic method, which is used to determine vibrational modes of molecules, although rotational and other low-frequency modes of systems may also be observed. Raman spectroscopy is commonly used in chemistry to provide a structural fingerprint by which molecules can be identified.

A Raman spectrum provides an instantaneous spectral coverage in a wide Raman shifts range to capture well-defined Raman peaks of the components present in the cell culture. These peaks are directly translated into important process information (i.e., concentrations, changes over time).

Raman spectroscopy allows for a discreet quantifiable compositional, chemical and physical properties of a sample real time on living cells in a non-destructive way within the fermenter without the need of any sample preparation, which are otherwise acquired via offline via assay measurements. Spectral preprocessing, along with multivariate analysis, such as PLS, PCA, ML, DL, ANN and/or IHM, for example, provides information that may be plotted on a time scale to inform and alert an operator, in real time, of the quantifiable and/or the relative status of important constituents during the bioprocess.

In all embodiments of the method according to the invention, a Raman spectrometer is applied for acquiring Raman spectra.

In one embodiment of the method, the concentration of the TauT inhibitor, selected from piperidine-4-sulfonic acid, g -Aminobutyric acid, g-vinyl-aminubutyric acid, β-alanine, or a pharmaceutically acceptable salt thereof, preferably piperidine-4-sulfonic acid or a pharmaceutically acceptable salt thereof is applied at a concentration of 0,5 and 100 mM, preferably between 0,5 and 10 mM.

In one embodiment of the method, the concentration of 5-ALA or a pharmaceutically acceptable salt thereof is applied at a concentration of 1 and 10 µg/mL.

In one embodiment, the method further comprises acquiring a mass spectrum of the cancer cell culture cells.

For analyzing the cell viability in a cell culture, state-of-the-art techniques are used.

One state-of the art technique is based on Trypan blue staining.

Trypan Blue, a diazo dye and a vital stain, has been used to selectively color dead tissues or cells blue. Live cells or tissues with intact cell membranes are not colored. Trypan Blue is not absorbed in a viable cell because cells are selective in the compounds that pass through the membrane. However, Trypan Blue traverses the membrane in a dead cell and stain dead cells with a distinctive blue color under a microscope.

Multi-sample cell count and viability analysis method commonly utilizes an image-based platform with automated liquid handling for mixing cell sample with Trypan Blue. The method uses one fixed focal plane to count live cells and Trypan Blue-stained dead cells and generates viability measurement.

Another state-of-the-art technique is flow cytometry. Flow cytometry is a technique used to detect and measure physical and chemical characteristics of a population of cells or particles.

In this process, a sample containing cells or particles is suspended in a fluid and injected into the flow cytometer instrument. The sample is focused to ideally flow one cell at a time through a laser beam, where the light scattered is characteristic to the cells and their components.

Cancer cells lines, which are used for the method include rat glioma cell lines (9L and C6), human glioma cell lines (U251 and T98G), human pancreatic cancer cell lines: Paca3 (wt TP53), MiaPaca2 (mutant TP53R248W), Panc1 (mutant TP53R273H), Panc02 (wt TP53), normal human pancreatic ductal epithelial cells (HPDE) immortalized by HPV transformation, human embryonic kidney 293 cells (HEK 293), Pig Kidney Epithelial Cells (LLC-PK1), MDCK (Madin-Darby Canine Kidney), Jurkat cells, Marine fibroblast, and MCF-7, EHEB (wt-p53) chronic B cell leukemia cells, HL-60 (p53-null) acute promyelocytic leukemia cell line, p53-defcient human colon HCT116 cancer cells, human non-small cell lung carcinoma cell line H1299, and osteosarcoma Saos2 cells.

In one embodiment of the method, for the above listed ells lines, which exclusively grow as adhesion cell culture, the cells are suspended in a buffer known in the art, such as PBS buffer, prior to analysis.

In one embodiment, the method further comprises:
(v) determining the amount of complex 1 consisting of PpIX/ mutant p53/ p73 complex and/or
(vi) determining the amount of complex 2 consisting of MDM2/mutant p53/p73 complex
(vii) calculating the ratio of complex 1 and complex 2, wherein
increased ratio of complex 1 to complex 2 is indicative of an antitumor effect of the composition of any one of the invention or an embodiment thereof or the composition for the use of the invention or an embodiment thereof.

Analysis of the formation of complexes in living cells is, e.g., carried out by fluorescent two-hybrid (F2H) Analysis in living cells. The rationale for the F2H assay is based on the fact that proteins are freely roaming the cell unless interactions with other cellular components immobilize them at specific structures. The method comprises tethering of fluorescently labeled MDM2 to the nucleus and subsequent analysis by confocal microscopy to assess complex formation as described by (Zolghadr et al., 2012, Methods Mol. Biol. 812:275-282).

Additional analysis of complex formation is carried out by other state-of-the-art techniques, such as chromatography, electron microscopy, immunoprecipitation in combination with Western Blot, and/or native PAGE.

In one embodiment, the method further comprises:
(v) applying analysis of the protein expression levels of levels of one or more than one target analytes, wherein the target analyte/s is/are selected from:
(a) Protoporphyrin IX (PpIX)
(b) active p73
(c) mutant p53
(d) reactive oxygen species
(e) platelets,
(g) taurine transporter inhibitor,
(h) taurine.

In several embodiments of the method, the method further comprises acquisition of an offline assay measurement as a reference Raman spectrum of each of the complex 1, complex 2, and any one of compounds (a)-(h) concurrently to acquiring Raman spectra of the cancer cell culture cell sample.

Based on the offline assay measurements, which are used for referencing purposes of the composition and respective analyte concentrations of the measured samples, a correlative model of one or more than one target analytes is generated as a training data set, such that spectral changes in the training data set correlate with the offline measurements of the target analyte properties and/or compositions.

After having acquired the offline measurement of the target analytes and the sample set, a correlative model to process the data set to qualitatively and/or quantitatively predict a value of a property and/or composition of the target analyte.

The physical samples for the offline assay measurements are acquired concurrently with the Raman spectra. Thereby, a direct correlation of the Raman measurement and the reference data acquired by an offline assay method is established.

In an additional embodiment, Raman spectra of each target analyte is acquired prior to an/or after the online Raman measurements.

The assay measurements are conventional methods known to the skilled person. These assays include, e.g., assays performed in vitro, such as immunoprecipitation, followed by Western-blot analysis, HPLC and Mass spectrometry, and qualitative and/or quantitative PCR analyses, wherein the methods may be applied alone or they are applied in combination, and cell-based assays for actual cells, such as fluorescent two-hybrid (F2H) analysis, automated cell counting comprising the determination of total cell count (TTC), and viable cell count (VCC), and the ratio of the aforementioned, wherein the measurement is based on Trypan-blue staining, or by fluorescence activated cell sorting (FACS).

In some embodiments of the method a Raman analyzer with a 785 nm laser is used, wherein the spectral coverage ranges between 150 - 3425 cm-1, and the resolution is 4 cm-1 or lower. In other embodiments of the method a Raman analyzer with a 993 nm laser is used (for comparison with the 785 nm one), wherein the spectral coverage ranges between 200-2400 cm-1, and the resolution is 5 cm-1 or lower. At 993 nm it is possible to reduce fluorescence in the Raman signal.

In one embodiment method is carried out continuously as a real-time analysis. In another embodiment, the Raman spectra are acquired at an interval, which lies between 1 minute and 2 hours, preferably between 1 minute and 20 minutes, more preferably between 1 minute and 5 minutes.

In some embodiments, the Raman spectra are acquired by a Raman system including a Raman probe, which is configured and arranged to transmit excitation light into the bioprocess vessel under test to collect a Raman signal emitted from a sample volume within the fermentation vessel. The Raman probe may be coupled to an excitation light source by a fiber optic cable such that excitation light emitted by the light source is incident upon a sample within the sample volume via a process connection. In at least one embodiment, the process connection includes an opening and is configured to enable attaching the probe to the fermentation vessel such that the probe is in direct contact with the sample via the opening of the process connection. In a further embodiment, the process connection may include a window that is transparent to the excitation light of the light source such that the probe is in indirect contact with the sample via the window of the process connection.

In some embodiments of the method, the Raman spectrum is carried out by a Raman flow assembly, comprising: The Raman Rxn-10 probe that transmits the Raman signal to the analyzer along an optical fiber. The micro flow cell that contains the cell culture and gives a significant amplification of the collected Raman signal, wherein the flow cell has a total volume of around 2-20 ml, preferably 4-18, more preferably 12-14 ml.

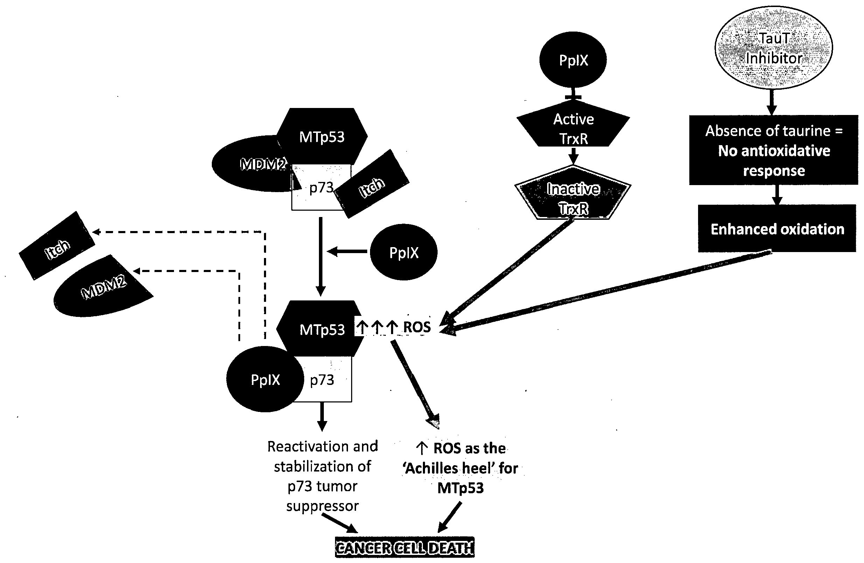

Fig. 1: Schematic representation of the interaction of MTp53/p737MDM2-complex and the mechanism of action by PpIX and a Taurine transporter inhibitor to disintegrate the complex .

Fig. 1 shows the course of biochemical reactions in a cell with a mutated p53. In a cell with a mutated p53 (MTp53), a complex between p73, MTp53, and MDM2 is formed. When PpIX is present in a cell it attaches to p73 and p53, and thereby disintegrates the complex formed between p73, MTp53, and MDM2. PpIX interaction also induces the detachment of E3 ubiquitin-protein ligase ITCH from p73. PpIX also fulfills a pro-oxidant function, by inhibiting thioredoxin reductase (TrxR) and elevating Reactive Oxygen Species (ROS) level for the induction of apoptosis (cell death) in cancer.

In summary, the mechanism of action of PpIX and TauT inhibitor leads to promoting cancer cell death.

Any one embodiment described herein is contemplated as being combinable with any one or more of the other embodiments, where applicable and not specifically disclaimed, even though the embodiments are described in different aspects of the present invention.

### List of reference symbols

- MTp53: Mutant tumor protein p53
- p73: tumor protein 73
- MDM2: Mouse double minute 2 homolog/ E3 ubiquitin-protein ligase Mdm2
- PpIX: Protoporphyrin IX
- TauT: Taurine transporter
- ROS: Reactive Oxygen Species
- TrxR: Thioredoxin reductase
- ITCH: E3 ubiquitin-protein ligase ITCH

## Claims

1. A composition comprising
(i) a taurine transporter (TauT) inhibitor, wherein preferably the TauT inhibitor is selected from
piperidine-4-sulfonic acid, g-Aminobutyric acid, g-vinyl-aminobutyric acid, β-alanine, or any pharmaceutically acceptable salt thereof
and
(ii) 5-aminolevulinic acid (5-ALA) or a pharmaceutically acceptable salt thereof.

2. The composition of claim 1, further comprising pharmaceutical carrier, wherein preferably the carrier is an adjuvant.

3. The composition of any one of claims 1-2 for use in treating cancer, wherein the composition is to be administered to a cancer patient to inhibit the growth of cancer cells.

4. The composition for the use of claim 3, wherein the TauT inhibitor, selected from piperidine-4-sulfonic acid, g-Aminobutyric acid, g-vinyl-aminobutyric acid, β-alanine, or any pharmaceutically acceptable salt thereof, preferably piperidine-4-sulfonic acid or a pharmaceutically acceptable salt thereof, and 5-aminolevulinic acid (5-ALA) or a pharmaceutically acceptable salt thereof are to be administered orally, wherein the administration of piperidine-4-sulfonic acid or a pharmaceutically acceptable salt thereof and 5-aminolevulinic acid (5-ALA) or a pharmaceutically acceptable salt thereof is separate, sequential, and/or simultaneous.

5. The composition for the use of claim 4, wherein the
TauT inhibitor, selected from piperidine-4-sulfonic acid, g-Aminobutyric acid, g-vinyl-aminobutyric acid, β-alanine, or any pharmaceutically acceptable salt thereof, preferably piperidine-4-sulfonic acid or a pharmaceutically acceptable salt thereof is to be administered to a human in a total amount of 500 mg, and
5-aminolevulinic acid (5-ALA) is to be administered to a human in an amount of 100 mg.

6. The composition for the use of claim 5, wherein selected from piperidine-4-sulfonic acid, g-Aminobutyric acid, g-vinyl-aminubutyric acid, β-alanine, or any pharmaceutically acceptable salt thereof, preferably piperidine-4-sulfonic acid or a pharmaceutically acceptable salt thereof is administered twice daily and 5-aminolevulinic acid (5-ALA) or a pharmaceutically acceptable salt thereof is administered in an amount of 100 mg once daily.

7. The composition for the use of claim 6, wherein the administration regimen is repeated for at least 2 months, preferably for at least 6 months.

8. The composition for the use of any one of claims 3-7, wherein the cancer cells are tumor cells have a mutation in the TP53 gene, wherein the mutation results in an altered expression of a p53 translation product and/or in expression of a non-functional p53 protein that corresponds to an alteration in a human p53 gene associated with tumor growth.

9. The composition for the use of any one of claims 3 to 8, wherein the cancer is a brain tumor, in particular, a glioblastoma, a pancreatic tumor, a kidney tumor, colon carcinoma, osteosarcoma, or wherein the cancer is leukemia.

10. A kit for analyzing anti-tumor effect of 5-aminolevulinic acid and/or taurine transporter (TauT) inhibitor comprising
(i) a Raman spectrometer
(ii) a cell imaging apparatus, and
(iii) a composition of claims 1-2 or a composition for the use of any one of claims 3-9.

11. An *in vitro* method for monitoring the efficacy of anti-tumor growth therapy of a composition according to any one of claims 1 to 2, wherein the method comprises:
providing tumor cells as a cell culture having a mutation in the TP53 gene, wherein the mutation results in an altered expression of a p53 translation product and/or in expression of a non-functional p53 protein that corresponds to an alteration in a human *p53* gene associated with tumor growth;
contacting the tumor cells with a taurine transporter (TauT) inhibitor, wherein the taurine transporter inhibitor is selected from
piperidine-4-sulfonic acid, g-Aminobutyric acid, g-vinyl-aminubutyric acid, β-alanine or any pharmaceutically acceptable salt thereof, preferably piperidine-4-sulfonic acid or a pharmaceutically acceptable salt thereof and/or
contacting the tumor cells with 5-aminolevulinic acid (5-ALA) or a pharmaceutically acceptable salt thereof
optionally contacting the tumor cells with an anti-cancer drug selected from a chemotherapeutic agent, and
and acquiring a Raman spectra of the cancer cell culture cell samples online.

12. The method of claim 11, wherein the method further comprises:
determining the viable cell count,
determining the total cell count and
calculating cell viability percentage based on viable cell count and total cell count.

13. The method of claim 12, wherein the method further comprises:
(v) determining the amount of complex 1 consisting of PpIX/ mutant p53/ p73 complex and/or
(vi) determining the amount of complex 2 consisting of MDM2/mutant p53/p73 complex
(vii) calculating the ratio of complex 1 and complex 2, wherein
increased ratio of complex 1 to complex 2 is indicative of an antitumor effect of the composition of any one of claims 1 to 2 or the composition for the use of any one of claims 3-9.

14. The method of any one of claims 11 to 14, wherein the method further comprises:
(v) applying analysis of the protein expression levels of levels of one or more than one of
(a) Protoporphyrin IX (PpIX)
(b) active p73
(c) mutant p53
(d) reactive oxygen species
(e) platelets,
(g) taurine transporter inhibitor,
(h) taurine.

15. The method of claims 14 or 15, wherein the method further comprises acquisition of offline assay measurements of each of the complex 1, complex 2, and any one of compounds (a)-(h) of claim 14, concurrently to acquiring Raman spectra of the cancer cell culture cell sample.

16. The method of claim 11, wherein the method is carried out continuously as a real-time analysis or wherein Raman spectra are acquired at an interval, which lies between 1 minute and 2 hours, preferably between 1 minute and 20 minutes, more preferably between 1 minute and 5 minutes.
